# EUROPEAN PATENT APPLICATION

(11) **EP 2 535 710 A1**
(43) Date of publication of application: **19.12.2012**
(21) Application number: 11169867.6
(22) Date of filing: 14.06.2011
(51) Int. Cl.: G01N 33/50

(54) **Method for determining the immune toxicity of a compound in vitro**

(71) Applicant: Rijksinstituut Voor Volksgezondheid En Milieu, 3721 MA Bilthoven (NL)
(72) Inventor: Osman, Ahmed Mohamud, 1273 CJ Huizen (NL); Van Loveren, Hendrik, 3734 CN Den Dolder (NL); Pennings, Jeroen Lambertus Antonius, 3562 TM Utrecht (NL)
(74) Representative: Habets, Winand

(57) **Abstract**

This invention is in the field of medical molecular diagnostics. It provides methods and means for the *in vitro* determination of immunotoxicity of a compound by measuring the phosphorylation levels and/or the levels of phosphoproteins in a cell exposed to a potentially immunotoxic compound. More in particular, the invention relates to an in vitro method for determining the immunotoxicity of a compound wherein a cell is exposed to the compound, and wherein the level of at least one phosphoprotein selected from the group consisting of Matrin3, MAPK1, RPS6KB1, DPYSL2, ACACA1 and RRM2 is determined in the exposed cell and wherein a level below a predetermined reference value of Matrin-3, RPS6KB1, MAPK1 or RRM2 or a level above the predetermined reference value of DPYSL2 or ACACA1 indicates that the compound is immunotoxic.

## Description

### Field of the invention

This invention is in the field of medical molecular diagnostics. It provides methods and means for the *in vitro* detection of the immunotoxicity of compounds by determining the phosphorylation status of a limited number of phosphoproteins.

### Background of the invention

Immunosuppression is the result of direct toxicity of compounds to components of the immune system. This kind of immunotoxicity causes malfunctioning of the immune system, with consequences such as reduced resistance to infections or immune dysregulation.

One of the best studied model compounds for immunotoxicity is tributyltin oxide (TBTO). Immunotoxicity is the most sensitive toxicological endpoint of TBTO. Most apparent is the direct toxicity towards cortical thymocytes resulting in thymus involution and peripheral lymphocyte depletion and thereby a diminished cellular immune response. TBTO-induced thymus atrophy is most likely explained by inhibition of cell proliferation or, alternatively, by induction of apoptosis in thymocytes.

Tributyltin oxide (TBTO) has been extensively used as a wood preservative. Tributyltin compounds are used as marine anti-biofouling agents and belong to the class of organotin compounds. Concerns over toxicity of these compounds (some reports describe biological effects to marine life at a concentration of 1 nanogram per liter) have led to a worldwide ban by the International Maritime Organization.

Organotin compounds or stannanes are chemical compounds based on tin with hydrocarbon substituents. Organotin chemistry is part of the wider field of organometallic chemistry.The first organotin compound was diethyltin diiodide, discovered by Edward Frankland in 1849. An organotin compound is commercially applied as a hydrochloric acid scavenger (or heat stabilizer) in polyvinyl chloride and as a biocide. n-Butyltin trichloride is used in the production of tin dioxide layers on glass bottles by chemical vapor deposition.

Tetraorganotins are very stable molecules with low toxicity and low biological activity. They are unusable as biocides, but they can be metabolized to toxic triorganotin compounds. They are used as starting materials for catalysts.

Triorganotins are very toxic. Tri-n-alkyltins are phytotoxic and therefore cannot be used in agriculture. Depending on the organic groups, they can be powerful bactericides and fungicides. Tributyltins are used as industrial biocides, e.g. as antifungal agents in textiles and paper, wood pulp and paper mill systems, breweries, and industrial cooling systems. Triphenyltins are used as active components of antifungal paints and agricultural fungicides. Other triorganotins are used as miticides and acaricides.

Diorganotins have no antifungal activity, low toxicity, and low antibacterial activity, except for diphenyltins. They are used in polymer manufacturing, as PVC heat stabilizers, catalysts, in the manufacturing of polyurethane and silicone curing. DBT is however immunotoxic, and a recent paper suggests a link to autoimmune related diseases.

Monoorganotins have no biocidal activity and their toxicity to mammals is very low. Methyltin, butyltin, octyltin and monoestertins are used as PVC heat stabilizers.

Many different organotin complexes are being studied in anticancer therapy, observing that their cytotoxicity and selectivity towards cancer cell is higher than that of cisplatin.

Detection of immunotoxicity of compounds is costly in terms of animals, time and money. Currently, the in vivo method used for immunotoxicity assay, which is based on the detection of KLH antibody, takes 28 days and the sacrifice of 40 animals for each compound.

Other, non-functional studied parameters of immunotoxicity include weight of lymphoid organs, histopathology, white blood cell counts and differentiation, and immunoglobulin levels in serum. In addition, activity of immune cells can be examined, e.g. macrophage activity, natural killer cell activity, and mitogen responses of spleen cells. In vitro methods are not yet accurate enough to serve as practical alternatives.

It is an object of the present invention to overcome or ameliorate at least some of the disadvantages of the prior art methods.

### Summary of the invention

It was found that six phosphoproteins involved in cell energy metabolism and proliferation became differentially phosphorylated after exposure to the model immunotoxic compound, TBTO. These six proteins are Matrin 3, RPS6KB1, DPYSL2, MAPK1, ACACA1, and RRM2.

This finding allows for the easy in vitro determination of the toxicity of other compounds. Hence, the invention relates to an in vitro method for determining the immunotoxicity of a compound wherein a cell is exposed to the compound, and wherein the level of at least one phosphoprotein selected from the group consisting of Matrin 3, MAPK1, RPS6KB1, DPYSL2, ACACA1 and RRM2 is determined in the exposed cell and wherein a level below a predetermined reference value of Matrin-3, RPS6KB1, MAPK1 or RRM2 and a level above the predetermined reference value of DPYSL2 or ACACA1 indicates that the compound is immunotoxic.

Because levels of phosphoproteins can be measured conveniently in a high-throughput system, a screening system based on these proteins may be used as a rapid compound screening system for immunotoxicity..

### Legend to the figure

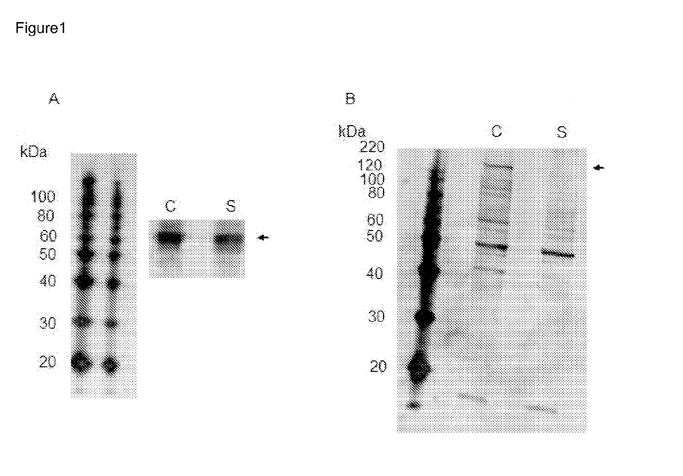

Figure 1:Panel A Immunoblot analysis of RPS6KB1 (Thr444/Ser447) and Panel B Matrin 3 expression levels obtained from TBTO-treated and control cells.

Molecular weight markers of ECL markers used ranged from 220-20 kDa.

### Detailed description of the invention

We found that the level of a number of 6 phosphorylated proteins in a cell or cell extract was indicative of the fact whether that cell had been exposed to an immunotoxic compound. When the levels of these 6 proteins in TBTO-exposed cells were compared with their levels in non-exposed cells we found considerable and significant differences. Table 1 lists the proteins involved and their phosphorylation sites.

Hence, the invention relates to an in vitro method for determining the immunotoxicity of a compound wherein a cell is exposed to the compound, and wherein the level of at least one phosphoprotein selected from the group consisting of Matrin3, MAPK1, RPS6KB1, DPYSL2, ACACA1 and RRM2 is determined in the exposed cell and wherein a level below a predetermined reference value of Matrin-3, RPS6KB1, MAPK1 or RRM2 and a level above the predetermined reference value of DPYSL2 or ACACA1 indicates that the compound is immunotoxic.

The predetermined reference value may be any suitable cut-off value that can be determined empirically by the skilled person. Preferably it is a value derived from the expression level of the same gene in a cell exposed to a control compound or no compound at all. Even more preferred is a reference value obtained from an average value of several independent experiments of cells exposed to a control compound or no compound at all. The skilled person is aware of the particulars of determining reference values for measuring and determining the levels of phosphoproteins.

In a preferred embodiment, the invention relates to a method wherein the level of all 6 marker phosphoproteins are determined. In this embodiment, the invention relates to a method as described above, wherein the level of phosphoproteins Matrin3, MAPK1, RPS6KB1, DPYSL2, ACACA1 and RRM2 is determined

The level of a protein in a cell extract or in a complete cell can be measured using standard techniques known in the art. For that purpose, immunoblotting may be a useful tool, but also immunohistochemistry may provide useful results. Antibodies against the proteins directed towards epitopes on the protein or towards specific phosphorylation sites may advantageously be employed.

Levels of these six phosphoproteins may also be detected using methods such as antibody microarrays, or traditional immunoassay-based analytical methods such as Western blotting or ELISA. For immunoassay methods, both general and phospho-site specific antibodies are commercially available in the market for these proteins.

Among the differentially phosphorylated proteins identified in the treated cells but not in control cells were key enzymes that catalyze rate-limiting steps in biosynthetic pathways. One of these enzymes was acetyl-CoA carboxylase isoform 1 (ACACA1), which catalyzes the rate-limiting step of fatty acid synthesis [25]. Phosphorylation sites at residues S79, S77 and S76 were identified for this protein (Table 1). The identification of two critical phosphorylation sites S77 and S79 (Table 1), known to inhibit acetyl-CoA carboxylase activity [25], indicate that this enzyme is inactive in cells exposed to immunotoxic compounds.

Among the differentially phosphorylated proteins identified in the control cells but not in treated cells were MATR3, RPS6KB1, MAPK1 and RRM2.

RPS6KB1, a downstream effecter of mTOR pathway and a major kinase for 40 ribosomal S6, whose phosphorylation is often associated with increase in protein synthesis and cell growth [29, 30 and the references therein], was preferentially present in control cells. Western blot analysis confirmed the decrease in the intensity signal of the band corresponding to Thr444/Ser447 of the treated cells as compared to that in the control extract (Figure 1a).]. This downregulation of RPS6KB1 in the treated cells could explain the reported inhibition of protein synthesis by TBTO [9, 10].

Another marker protein is MAPK1, known also as extracellular signal-regulated kinase, which has essential roles in cell proliferation, growth and differentiation [31]. Phosphorylation sites at T179, T183, T188, and Y185 were identified for this protein in control cells (Table 1). This protein was not detected in TBTO-treated cells whereas it was abundantly present in control cells not exposed to TBTO.

Another phosphoprotein that was downregulated in TBTO-cells was Matrin 3 (Table 1). This nuclear matrix protein has been implicated in replication, transcription and RNA processing [34, and 35 and the references therein]. Phosphorylated sites at the residues S596, Y597, S188 and Y526 were identified only in the control cells (Table 1). Immunoblot experiments showed that only one band corresponding to the apparent molecular weight of Matrin 3 (ca. 125-130 kDa) was observed in control extract but not in TBTO-treated sample (Fig. 1 b). It has been previously shown that Matrin 3 mediates neuronal death following NMDA activation of rat neurons and that this protein acts as a substrate of cAMP-dependent protein kinase, whose phosphorylations leads to degradation of matrin 3 [38]. A recent study has shown that Matrin 3 is a downstream substrate of caspases 3 and 8 [34]. Interestingly, both cAMP-dependent kinase and caspase 3 were identified in TBTO-treated cells (not shown). This might explain why the band corresponding to Matrin 3 was not detectable in TBTO-treated cells (Fig. 1 b).

Another marker phosphoprotein identified in the control cells is ribonucleotide reductase, subunit RRM2. This protein has been shown to be present in late G1/early S phase of the cell cycle, contrary to the other subunit, RRM1, whose activity is stable throughout the cell cycle [32, 33]. For this protein the phosphorylation site S20 was identified (Table 1). Earlier study showed that RRM2 is phosphorylated at the S20 site by p34Cdc2 kinase [49]. Using mutagenesis, the Wnt gene assay and RNA interference, Tang et al. [50] provided evidence that RRM2 may act as inhibitor of β-catenin, a downstream effecter of Wnt signaling pathway and that phosphorylation at S20 may relieve of this inhibition. In other words, in addition to DNA synthesis, RRM2 upon phosphorylation at S20 decreases the threshold of β-catenin effect; hence increasing the latter's transcriptional effect on target genes. Our results suggest that toxic organotin compounds not only affect cell proliferation by inducing apoptosis as described before [9, 10], but could also, at lower concentrations, affect the rate of cell cycle.

**Table 1**

| **Gene Symbol** | **Protein Name** | **Accession number** | **Sequences of the phosphopeptides identified** | **Phosphorylation sites** | **Present in treated cells** | **Present in control cells** |
|---|---|---|---|---|---|---|
| Matr3 | Matrin-3 | IPI00453826.2 | R.pSYSPDGKESPSDKK.S | S596 | - | + |
| | | | R.SpYSPDGKESPSDKK.S | Y597 | - | + |
| | | | R.RDpSFDDRGPSLNPVLDYDHGSR.S | S188 | - | + |
| | | | K.IKNpYILMRMXK.S | Y526 | - | + |
| | | | R.TEEGPTLpSpYGRDGR.S | S157, Y158 | - | + |
| | | | | | | |
| RPS6KB1 | Isoform Alpha I of Ribosomal protein S6 kinase beta-1 | IPI00453603.1 | R.pTPVSPVKFSPGDFWGR.G | T444 | - | + |
| | | | R.TPVSPVKFpSPGDFWGR.G | S452 | - | + |
| | | | | | | |
| DPYSL2 | Dihydropyrimi dinase-related protein 2 | IPI00114375.2 | K.pTVTPASSAKTSPAK.Q | T514 | + | - |
| | | | K.TVTPASSAKpTSPAK.Q | T521 | + | |
| | | | K.TVTPASpSAKTSPAK.Q | S518 | + | - |
| | | | K.TVTPApSSAKTSPAK.Q | S517 | + | - |
| | | | | | | |
| MAPK1 | Mitogen-activated protein kinase 1 | IPI00119663.3 | R.VADPDHDHTGFLpTEpYVATR.W | T183, Y185 | - | + |
| | | | R.VADPDHDHTGFLpTEYVApTR.W | T183, T188 | - | + |
| | | | R.VADPDHDHTGFLTEpYVApTR.W | Y185, T188 | - | + |
| | | | R.VADPDHDHpTGFLTEpYVATR.W | T179, Y185 | - | + |
| | | | R.VADPDHDHTGFLpTEYVATR.W | T183 | - | + |
| | | | R.VADPDHDHTGFLTEpYVATR.W | Y185 | - | + |
| | | | | | | |
| ACACA1 | Isoform 1 of Acetyl-CoA carboxylase 1 | IPI00474783.4 | R.SSMpSGLHLVK.Q | S79 | + | - |
| | | | R.SpSMSGLHLVK.Q | S77 | + | - |
| | | | R.pSSMSGLHLVK.Q | S76 | + | - |
| | | | | | | |
| Rrm2 | Ribonucleosid e-diphosphate reductase subunit M2 | IP100112645.1 | R.TPLATIADQQQLQLpSPLKR.L | S20 | - | + |

### Examples

### Example 1: Chemicals

Bovine beta-casein, α-casein, bovine serum albumin, ovalbumin, TBTO, DL-dithiothreitol, and iodoacetamide were purchased from Sigma. Sequencing-grade modified trypsin was from Roche (Mannheim, Germany). ReproSil-Pur18-AQ3 resin was obtained from Dr. Maisch GmsH, (Ammerbuch, Germany) and C18 200A-AQ5 was purchased from Phenomenex. Ammonium bicarbonate was obtained from Fluka. The solvents ethanol absolute and acetonitrile (HPLC grade) were purchased from Bissolve. The gel for electrophoresis (10% NU-PAGE Bis-Tris-gel), Nu-PAGE-SDS Running buffer (20X), Transfer buffer (20X), LDS (lithium dodecyl sulfate) sample buffer (4X) and Nu-PAGE Antioxidant agent were obtained from Invitrogen Company. Anti-matrin 3 goat polyclonal IgG (primary anti-body) and donkey anti-goat IgG-HRP, anti-p70 S6 kinase (Thr 421/Ser424)-R rabbit polyclonal antibody were purchased from Santa Cruz Biotechnology. Hypond-P polyvinylidene difluoride (PVDF) membrane, ECL molecular weight markers (Magic standard) was from Invitrogen and ECL detection reagents were obtained from Amersham Biosciences, UK. Phosphatase Cocktail Inhibitor 1 was from Sigma, PhosphoProtein Purification Kit was obtained from Qiagen. MonoTip Tio pipette tips (200 µL)) were purchased from GL Sciences (Darmstadt, Germany) and Amicon Ultra membrane 0.5 mL 3K was obtained from Millipore (Carrigtwahill, Ireland).

### Example 2: Cell culture and treatment with TBTO

Murine EL4 cells were cultured as described before [10]. Each treated sample or control contained 5×107 cells in a volume of 20 mL. A 1 mM TBTO stock solution was prepared in ethanol and 10 µl of this solution was added to each one of the cell suspension samples so that the final concentration of TBTO was 0.5 µM, whereas 10 µl of ethanol without TBTO was added to the control cells. Two independent replicate experiments for both treated sample and control were performed. Subsequently, cells were incubated at 37 °C for 6 hr. Cell viability was evaluated by trypan blue dye exclusion and ranged 85-90% in all experiments, which was in agreement with previous reports [10, 21]. TBTO Concentrations higher than 1µM and incubation times longer than 6h caused apoptosis of cells [10, 21].

### Example 3: Protein Extraction and Phosphoprotein Enrichment Procedure

After exposure, cell suspensions were centrifuged at 1200 rpm for 10 min at 4 °C and the pellets were washed in 20 mL phosphate-buffered saline (PBS, pH 7.2) and centrifuged again. This procedure was repeated twice. The obtained cell pellet of each sample or control was then suspended in 5 mL of phosphoprotein lysis buffer prepared following the instructions of Phosphoprotein Purification Kit (Qiagen). This volume of the lysis buffer contained, in addition to the detergent CHAPS and Benzonase (10 µL), one tablet of protease inhibitor and Phosphatase Inhibitor Cocktail 1 (10 µL). The cell suspension was incubated on ice for 30 min and vortexed briefly and gently every 10 min. After that, the cell lysate was centrifuged at 10.000 rpm for 30 min at 4 °C. The supernatant was then taken and protein concentration determined according to Bradford [22]. A total amount of 2.5 mg protein, at the concentration of 0.1 mg/mL, was loaded on the affinity column, which was pre-equilibrated with lysis buffer as recommended by the manufacturer. The chromatography was performed at room temperature. After the column was washed with the remaining lysis buffer, the enriched bound fractions of phosphoproteins were eluted using the phosphoprotein elution buffer, accompanying the Kit. These enriched fractions were combined and the protein concentration was determined. The combined fractions were dialyzed to remove CHAPS and concentrated using Amicon Ultra 0. 5mL, 3K cut off. The Ultra-filtrate was reduced to circa 50 µL and used for electrophoresis.

### Example 4: Gel-electrophoresis and In-gel digestion

Electrophoresis of the samples was performed essentially as described in detail before [10]. Briefly, 80 µg of protein was loaded per well of a 10% NU-PAGE-Tris-gel and run at 160 V for 90 min. After that, the gel was rinsed 3 times with deionized water (5 min each time), followed by staining it with Coomassie (Simply Blue Safe Stain) for 2 hrs. The gel lanes of sample and control were horizontally cut into 14 fractions. Each gel slice was cut into pieces and put into a 1.5 mL eppendorf tube.

The obtained fractions were destained, dehydrated, reduced with DTT and alkylated with Iodoacetamide as described before [10]. The fractions were digested with trypsin and incubated for 17 hr. After that, to each fraction 30 µL of ammonium bicarbonate buffer was added and incubated for additional 2 hr. The supernatant of each fraction was put into an eppendorf tube and subsequently 25 µL of 50% ethanol containing 5% formic acid was added to each fraction, mixed and pooled it to the previous one. This process was repeated once. The obtained fractions were freeze-dried by Speed-Vac, suspended in 60 µL acetonitrile and again freeze-dried. Finally, the peptide fractions were suspended in 50 µL solution of 0.5% formic acid/2% acetonitrile and used for phosphopeptide enrichment.

### Example 5: Titanium dioxide enrichment of phosphopeptides

Phosphopeptides were enriched from peptide mixture obtained as described above using titanium dioxide MonoTip Tio pipette tips. Essentially, the operation protocol of the manufacturer was followed with slight modification. The Monotip Tio was preconditioned with 100% acetonitrile and this process was repeated twice. Next, the tip was conditioned with 0.2 M phosphate buffer, pH 7.0 and equilibrated with 50% aqueous acetonitrile solution containing 0.1% formic acid, repeating this step thrice. After that, peptide fraction was drawn and ejected. This adsorption process was repeated 25 cycles per fraction, after which the tip was rinsed with 30% aqueous acetonitrile containing 0.1 % formic acid and 0.1 M KCI. The rinsing process was repeated 6 times per fraction. Finally, the tip was desalted with the equilibration buffer, drawing and ejecting three times, followed by elution with 2% aqueous ammonia. The eluate fractions were freeze-dried, suspended with 60 µL of 100% acetonitrile per fraction and again freeze-dried. After that, each fraction was suspended with 30 µL of 1% formic acid/2% acetonitrile aqueous solution. The fractions were kept at -20 °C until analysis.

### Example 6: Nano-LC/MS/MS

Peptide fractions were analyzed by a nano-LC-MS/MS using an Agilent 11000 Series LC set up (vacuum degasser, autosampler and one high pressure-mixing binary pump without static mixer) coupled to a LCQ Deca Quadruple Ion Trap mass spectrometer (Thermo Finningan, San Jose, CA) as previously described by Meiring et al. [23] The conditions of elution were essentially as described in [10]. Briefly, we loaded 5 µL of peptide solution to a trap column (Aqua C18 [Phenomenex]; I = 15 mm-100 µm ID, packed in house) at 5 µ/min of 100% Solvent A (0.1 M acetic acid). Following a decrease of the flow to 150 nl/min by a splitter, peptides were transferred to Reprosil C18 RP column (Dr. Maisch GmbH (I= 20 cm, 50 µm ID) with a linear gradient from 0-100% Solvent B (0.1 M acetic acid in 80% acetonitrile) in 40 min. Solvent B was maintained at 100% for 5 min, followed by decreasing solvent B to 0% in 0.1 min, washing and re-equilibrating the system at 100% A for 10 min. The LCQ operated in the data-dependent mode using a positive ion mode. Full MS spectra from m/z 400 to 2000 were acquired followed by a tandem spectra of the three most intense precursor ions present in the MS scan. Other instrument parameters have been previously described [10].

### Example 7: Database searches

All MS/MS data were analyzed using SEQUEST engine (Bioworks 3.3 version) [24] and Proteome Discoverer (version 1.1) against the IPI mouse 3.69 fasta database. A mass tolerance of 1.4 for the precursor and 1 Da for mass fragments were applied. Trypsin was used for protein digestion, allowing 2 missed cleavages, static modification for cysteine (carbamiodomethylation) and variable phosphorylation of Ser/Thr/Tyr and methionine oxidation. The following filtering threshold criteria were used: a Delta Cn (Δcn) was set to 0.08, Sequest X corr vs. charge state (xcorr = 2 for z= 1, Xcorr = 1.5 for 2 and Xco r = 2.5 for z = 3).

### Example 8: Western blot analysis

Cell extracts for immunoblotting were prepared as described above. Equal amounts of protein (60 µg) from TBTO-treated and non-treated cells were separated by SDS-PAGE, followed by electrotransfer of the proteins using PVDF (Hybond-P polyvinylidene difluoride) membrane at 25 V for 90 min. The membrane was blocked by using non- fat milk powder in PBS containing 0.2% Tween and probed with goat anti-matrin-3 antibody or with rabbit anti-p70 S6 kinase (Thr421/Ser424) antibody. Detection was performed by using donkey anti-goat IgG-HRP and goat anti-rabbit IgG-HRP, respectively, and ECL Western blotting detection reagents (Amersham Pharmacia).

### References

1. Schrantz, J. 1990. TBTO marine paint under EPA scrutiny. Ind. Finishing 66: 58-61.
2. Elsabbagh, H. S., Moussa, S. Z., EI-Tawil, O. S. 2002. Neurologic Sequelae of tributltin intoxication in rats. Pharm 45(3)201-206.
3. Kannan, K., Corsolini, S., Focardi, S., Tanabe, S., and Tatsukawa, R. 1996. Accumulation pattern of butyltin compounds in dolphin, tuna, and shark collected from Italian coastal waters. Arch. Environ. Contam. Toxicol. 35: 64-69.
4. Snoeij, N.J., Penninks, A. H., and Seinen, W. 1988. Dibutyltin and tributyltin compounds induce thymus atrophy in rats due to a selective action on thymic lymphoblasts. Int. J. Immunopharmacol. 10:891-899.
5. Vos, I. G., De Klerk, A., Krajnc, E. I., van Loveren, H., and Rozing, J. 1990. Immunotoxicity of bis(tri-n-butyltin)oxide in the rat: effects on thymus dependent immunity and on non-specific resistance following long term exposure in young versus aged rats. Toxicol. Appl. Pharmacol. 105: 144-155.
6. Van Loveren, H., Krajnc, E. I., Rombout, P. J., Blommaert, E. A., and Vos, J.G., 1990. Effects of ozone, hexachlorobenzene, and bis(tri-n-butlytin)oxide on natural cell killer activity in the rat lung. Toxicol. Appl. Pharmacol. 102:21-33.
7. Aluoch, A. O., Odman-Ghazi, S. O., and Whalen, M.M. 2006. Alteration of an essential NK cell signaling pathway by low doses of tributyltin in human natural killer cells. Toxicology 224:229-237.
8. Iguchi, T., Katsu, Y., Horiguchi, T., Watanabe, H., Blumberg, B., Ohta, Y. 2007. Endocrine disrupting organotin compounds are potent inducers of imposex in gastropods and adipogenesis in vertebrates. Mol. Cell. Toxicol. 3 (1):1-10.
9. Raffray, M., Mccarthy, D., Snowden, R., Cohen, G. M. 1992. Apoptosis as a mechanism of tributyltin cytoxicity to thymocytes: Relationship of apoptotic markers to Biochemical and cellular effects. Toxicol.Appl. Pharmacol 119: 122-130.
10. Osman, A. M., van Kol, S., Peijenburg, A., Blokland, M., Pennings, J. L. A., Kleijans, J. C.S., van Loveren, H. 2009. Proteomic analysis of mouse thymoma cells treated with bis(tri-n-butyltin)oxide (TBTO). J. Immunotoxicol 6(3):174-183.
11. Penninks, A. H., Verschuren, P. M., and Seinen, W. 1983. Di-n-butyltin dichloride uncouples phosphorylation in rat liver mitochondria. Toxicol. Appl. Pharmacol. 70:115-120.
12. Veiga, A., Pinto, A. F., Loureiro-Dias, M. C.1996. Tributyltin oxide affects energy production in the yeast Rhodotorula ferulica, a utilizer of phenolic compounds. Can. J. Microbiol. 43:683-687.
13. Soracco, R. J., and Pope D. H. 1983. Bacteriostatic and bactericidal modes of action of bis(tributyltin)oxide on Legionella penumophila. Appl. Environ. Microbiol. 45(1):48-57.
14. Jensen, K. G., Önfelt, A., Wallin, M., Andresen, O. 1991. Effects of organotin compounds on mitosis, spindle structure, toxicity and in vitro microtubule assembly. Mutagenesis 6 (5): 409-416.
15. Tan, L.P., Ng, M. L., and Kumar Das, V. G. 1978. The effect of trialkyltin compounds on tubulin polymerization. Neurochemistry 31, 1035-1041.
16. Baken, K. A., Pennings, J.L. A., de Vries, T.M., van Steeg, H., and van Loveren, H. 2006. Gene expression profiling of bis(tri-n-butyltin)oxide(TBTO)-induced immunotoxicity in mice and rats. J. Immunotoxicol. 3:227-244.
17. Baken, K. A., Arkusz, J., Pennings, J. L.A., Vandebriel, R.J., and van Loveren, H. 2007. In vitro immunotoxicity of bis(tri-n-butyltin)oxide (TBTO) studied by toxigenomics. Toxicology 237: 35-48.
18. Sburlati, A. R., Manrow, R. E., and Berger, S. I. 1991. Prothymosin-α antisense oligomers inhabit myeloma cell division. Proc. Natl. Acad. USA 88:253-257.
19. Evasfieva, A. G., Belov, G. A., Kalkum, M., Chichova, N. V., Bogdanov, A. A. Agol, V. I., and Vartapetian, A. B. 2000. Prothymosin-α fragmentation in apoptosis. FEBS Lett. 467:150-154.
20. Mann, M., Ong, S-E, Grøborg, M., Steen, H., Jensen, O. N.2000. Analysis of protein phosphorylation using mass spectrometry: deciphering the phosphoproteome. Trends Biotechnol 20(6):261-268.
21. Raffray, M., and Cohen, G. M., Bis(tri-n-butlytin)oxide induces cell death apoptosis in immature rat thymocytes. Arch.Toxicol. 65:135-139.
22. Bradford, M.M. 1976. A rapid and sensitive method for the quantitation of microgramquan tities of protein utilizing the principles of protein dye binding. Anal. Biochem. 72:248:254.
23. Meiring,H.D.,vanderHeeft,E.,TenHove,G.I.,andDeJong,A.P.2002.NanoscaleLC-Ms(n): Technical design and applications to peptide and protein analysis.J.Sep.Sci.25:5 57-568.
24. Yates,J.R.,Eng.J.K.,McCormack,A.L.,and Schultz, D., 1995. Method to correlatet and emmassspectra of modified peptides to a min oacid sequences in the protein data base.Anal.Chem.67:1426-1436.
25. Ha,J.,Daniel,S.,Broyles,S.S.,andKim,Ki-Han.1994.CriticalphosphorylationsitesforAcetyl-CoAcarboxylaseactivity.J. Biol. Chem.269(35):22162-22168.
26. Eguchi,S.,Oshiro,N.,Miyamoto,T.,Yoshino,K-I,Okamoto,S.,Ono,T.,Kikkawa,U.,Yonezawa,K.2009.AMP-activatedproteinkinasephosphorylatesglutamine:fructose-6-phosphateamidotransferase1atSer243tomodulateitsenzymaticactivity.GenesCells 14:179-189.
27. Linn,T.C,Pettit,F.H.,Reed,I.J.Alpha-ketoaciddehydrogenasecomplexes.X.Regulationoftheactivityofthepyruvatedehydr ogenasecomplexfrombeefkidneymitochondriabyphosphorylationanddephosphoryl ation.Proc.Natl.Acad.Sci.USA1969.62:234-241.
28. Rardin,M.J.,Wiley,S.E.,Naviaux,R.K.,Murphy,A.N.,Dixon,J.E.2009.Monitoringphos phorylationofthepyruvatedehydrogenase.Anal.Biochem.389(2):157-164.
29. Fingar,D.C.,andBlenis,J.2004.Targetoframpycin(TOR):anintegratorofnutrientandgr owthfactorsignalsandcoordinatorofcellgrowthandcellprogression.Oncogene23:315 1-3171.
30. Zhou,X.,Lin,D.S.,Zheng,F.,Sutton,M.A.,Wang,H.2010.Intracellularcalciumandcalm odulinlinkbrain-derivedneurotrophicfactortop70S6kinasephosphorylationanddendriticproteinsynth esis.J.Neurosci.Res.88(7):1420-1432.
31. Warren,D.T.,Tajsic,T.,Mellad,J.A.,Searles,R.,Zhang,Q.,Shanahan,C.M.2009.Nove Inulearnesprin-2variantstetheractiveextracellularsignal-regulatedMAPK1andMAPK2atpromyelocyticleukemiaproteinnuclearbodiesandactt oregulatesmoothmusclecellproliferation.J.Biol.Chem.285(2)1311-1320.
32. Engström,Y.,Eriksson,S.,Jildevik,I.,Skog,S.,Thelander,L.,andTribukait,B.1985.Cell cycle-dependentexpressionofmammalianribonucleotidereductase.260(16)9114-9116.
33. Heidel,J.D.,Liu,J.Y-C,Yen,Y.,Zhou,B.,andHeale,B.S.E.2007.PotentsiRNAinhibitorsofribonucleotidered uctasesubunitRRM2reducecellproliferationinvitroandinvivo.Clin.CancerRes.13:220 7-2215.
34. Valencia,C.,Ju,W.,Liu,R.2007.Matrin3isaCa+2/calmodulin-bindingproteincleavedbycaspases.Biochem.Bipphys.Res.Commun.361:281-286.
35. Erazo,A.,Yee,M.B.,Banfield,B.W.,andKinchington,P.R.2011.Thealphaherpesvirus US3/ORF66proteinkinasesdirectphosphorylationofthenuclearmatrixproteinmatrin3. J.Virol.85(1)568-581.
36. Alexandra,N.S.,Meijne,M.L.,vanderPol,A.J.,deJong,L.1990.Thenuclearmatrixfrom cellsofdifferentorigin.Evidenceforacommonsetofmatrixproteins.J.Biol.Chem.265(1 0):5460-5465.
37. Nakasu,H.,andBerezney,R.1991.Nuclearmatrins:Identificationofthemajornuclearm atrixproteins.Proc.Natl.Acad.Sci.USA88:10312-10316.
38. Giordano,G.,Pérez,A.M.S.,Montoliu,C.,Berezney,R.,Malavantham,K.,Costa,L.G.,C alvete,C.J.,Felipo,V.2005.ActivationofNMDAreceptorsinducesproteinkinaseA-mediatedphosphorylationanddegradationofmatrin3.Blockingtheseeffectsprevents NMDA-inducedneuronaldeath.J.Neurochem.94:808-818.
39. Hibino,Y.T.,Usui,Y.,morita,N.,Hirose,M.,Okazaki,M.S.,andHiraga,K.2006.Molecula rpropertiesandintracellularlocalizationofratlivernuclearscaffoldproteinP130.Biochi m.Biophys.Acta1759:195-207.
40. Bernert,G.,Fountoulakis,M.,andLubec,G.2002.Manifolddecreasedproteinlevelsofm atrin3,reducedmotorproteinHMPandhlarkinfetalDown'ssyndromebrainProteomics2 :1752-1757.
41. Macrae,T.H.1997.Tubulinpos-translationalmodifications.Eur.J.Biochem.244,265-278.
42. Aldridge,W.N.1958.Thebiochemistryoforganotincompounds.Biochem.J.69:367-376.
43. Corton,J.M.,Gillespie,J.G.,andHardie,D.G.1994.RoleoftheAMP-activatedproteinkinaseinthecellularstressresponse.CurrBiol.4:315-324.
44. Colliar,L.,Sturm,A.,andLeaver,M.J.2011.Tributyltinisapotentinhibitorofpiscineperox isomeproliferators-activatedreceptorαandβ.Comp.Biochem.Physiol.C.153:168-173.
45. Campbell,F.M.,Kozak,R.,Wagner,A.,Altarejos,J.Y.,Dyck,J.R.B.,Belke,D.D.,Severs on,D.L.,Kelly,D.P.,Lopaschuk,G.D.2002.Aroleforperoxisomeproliferator-activatedreceptora(PPARα)inthecontrolofcardiacmalonyl-CoAlevels.J.Biol.Chem.277(6):4098-4103.
46. Jager,S.,Handschin,C.,Pierre,J.S.,Speegelman,B.M.2007.AMP-activatedproteinkinase(AMPK)actioninskeletalmuscleviadirectphosphorylationofP GC-1α.Proc.Natl.Acad.Sci.USA104(29):12017-12022.
47. Shaw,R.J.,Lamia,K.A.,Vasquez,D.,Koo,S.h.,Bardeesy,N.,Depinho,R.A.,Montiminy ,M.,andCantley,L.C.2005.ThekinaseLKB1mediatesglucosehomeostasisinliverandt herapeuticeffectsofmetformin.Science310:1642-1646.
48. Leff,T.2003.AMP-activatedproteinkinaseregulatesgeneexpressionbydirectphosphorylationofnuclear proteins.Biochem.Soc.Trans.31:224-227.
49. Chan,A.K.,Persad,S.,Litchfield,D.W.,andWright,J.A.(1999).Ribonucleotidereducta seR2proteinisphosphorylatedatS-20byP34cdc2kinase. Bioch im. Biophys.Acta 1448:363-371.
50. Tang,LY,Deng,N.,Wang,LS,Dai,J.,Wang,ZL,Jiang,XS,Li,SJ,Li,L.,Li,L.,Sheng,QH, Wu,DQ,Li,L.,andZeng,R.2007.QuantitativephosphoproteomeprofilingofWnt3a-mediatedsignalingnetworkindicatingtheinvolvementofribonucleosidediphosphatere ductaseM2subunitphosphorylationatresidueserine20incanonicalWntsignaltransdu ction.Mol.CellProteomics6:1952-1967.

## Claims

1. In vitro method for determining the immunotoxicity of a compound wherein a cell is exposed to the compound, and wherein the level of at least one phosphoprotein selected from the group consisting of Matrin3, MAPK1, RPS6KB1, DPYSL2, ACACA1 and RRM2 is determined in the exposed cell and wherein a level below a predetermined reference value of Matrin-3, RPS6KB1, MAPK1 or RRM2 or a level above the predetermined reference value of DPYSL2 or ACACA1 indicates that the compound is immunotoxic.

2. Method according to claim1 wherein the level of phosphoproteins Matrin3, MAPK1, RPS6KB1, DPYSL2, ACACA1 and RRM2 is determined.


SEQUENCE LISTING
<110>  RIVM

<120>  METHOD FOR DETERMINING THE IMMUNOTOXICITY OF A COMPOUND IN VITRO
<130>  196 EPP0

<160>  21
<170>  PatentIn version 3.5

<210>  1
<211>  16
<212>  PRT
<213>  mus musculus

<220>
<221>  MOD_RES
<222>  (2)..(2)
<223>  PHOSPHORYLATION
<400>  1
Arg Ser Tyr Ser Pro Asp Gly Lys Glu Ser Pro Ser Asp Lys Lys Ser
1               5                   10                  15

<210>  2
<211>  16
<212>  PRT
<213>  mus musculus

<220>
<221>  MOD_RES
<222>  (3)..(3)
<223>  PHOSPHORYLATION
<400>  2
Arg Ser Tyr Ser Pro Asp Gly Lys Glu Ser Pro Ser Asp Lys Lys Ser
1               5                   10                  15

<210>  3
<211>  24
<212>  PRT
<213>  mus musculus

<220>
<221>  MOD_RES

<222>  (4)..(4)
<223>  PHOSPHORYLATION
<400>  3
Arg Arg Asp Ser Phe Asp Asp Arg Gly Pro Ser Leu Asn Pro Val Leu
1               5                   10                  15

Asp Tyr Asp His Gly Ser Arg Ser
            20

<210>  4
<211>  13
<212>  PRT
<213>  mus musculus

<220>
<221>  MOD_RES
<222>  (5)..(5)
<223>  PHOSPHORYLATION
<220>
<221>  misc_feature
<222>  (11)..(11)
<223>  Xaa can be any naturally occurring amino acid
<400>  4
Lys Ile Lys Asn Tyr Ile Leu Met Arg Met Xaa Lys Ser
1               5                   10

<210>  5
<211>  16
<212>  PRT
<213>  mus musculus

<220>
<221>  MOD_RES
<222>  (9)..(10)
<400>  5
Arg Thr Glu Glu Gly Pro Thr Leu Ser Tyr Gly Arg Asp Gly Arg Ser
1               5                   10                  15

<210>  6
<211>  18
<212>  PRT
<213>  mus musculus

<220>
<221>  MOD_RES
<222>  (2)..(2)
<223>  PHOSPHORYLATION
<400>  6
Arg Thr Pro Val Ser Pro Val Lys Phe Ser Pro Gly Asp Phe Trp Gly
1               5                   10                  15

Arg Gly

<210>  7
<211>  18
<212>  PRT
<213>  mus musculus
<400>  7
Arg Thr Pro Val Ser Pro Val Lys Phe Ser Pro Gly Asp Phe Trp Gly
1               5                   10                  15

Arg Gly

<210>  8
<211>  16
<212>  PRT
<213>  mus musculus

<220>
<221>  MOD_RES
<222>  (11)..(11)
<223>  PHOSPHORYLATION
<400>  8
Lys Thr Val Thr Pro Ala Ser Ser Ala Lys Thr Ser Pro Ala Lys Gln
1               5                   10                  15

<210>  9
<211>  16
<212>  PRT
<213>  mus musculus

<220>
<221>  MOD_RES
<222>  (2)..(2)
<223>  PHOSPHORYLATION
<400>  9
Lys Thr Val Thr Pro Ala Ser Ser Ala Lys Thr Ser Pro Ala Lys Gln
1               5                   10                  15

<210>  10
<211>  16
<212>  PRT
<213>  mus musculus

<220>
<221>  MOD_RES
<222>  (8)..(8)
<223>  PHOSPHORYLATION
<400>  10
Lys Thr Val Thr Pro Ala Ser Ser Ala Lys Thr Ser Pro Ala Lys Gln
1               5                   10                  15

<210>  11
<211>  16
<212>  PRT
<213>  mus musculus

<220>
<221>  MOD_RES
<222>  (7)..(7)
<223>  PHOSPHORYLATION
<400>  11
Lys Thr Val Thr Pro Ala Ser Ser Ala Lys Thr Ser Pro Ala Lys Gln
1               5                   10                  15

<210>  12
<211>  21
<212>  PRT
<213>  mus musculus

<220>
<221>  MOD_RES
<222>  (14)..(14)

<223>  PHOSPHORYLATION
<220>
<221>  MOD_RES
<222>  (16)..(16)
<223>  PHOSPHORYLATION
<400>  12
Arg Val Ala Asp Pro Asp His Asp His Thr Gly Phe Leu Thr Glu Tyr
1               5                   10                  15

Val Ala Thr Arg Trp
            20


<210>  13
<211>  21
<212>  PRT
<213>  mus musculus


<220>
<221>  MOD_RES
<222>  (14)..(14)
<223>  PHOSPHORYLATION
<220>
<221>  MOD_RES
<222>  (19)..(19)
<223>  PHOSPHORYLATION
<400>  13
Arg Val Ala Asp Pro Asp His Asp His Thr Gly Phe Leu Thr Glu Tyr
1               5                   10                  15

Val Ala Thr Arg Trp
            20


<210>  14
<211>  21
<212>  PRT
<213>  mus musculus


<220>
<221>  MOD_RES
<222>  (19)..(19)
<223>  PHOSPHORYLATION
<220>

<221>  MOD_RES
<222>  (19)..(19)
<223>  PHOSPHORYLATION
<400>  14
Arg Val Ala Asp Pro Asp His Asp His Thr Gly Phe Leu Thr Glu Tyr
1               5                   10                  15

Val Ala Thr Arg Trp
            20

<210>  15
<211>  21
<212>  PRT
<213>  mus musculus

<220>
<221>  MOD_RES
<222>  (10)..(10)
<223>  PHOSPHORYLATION
<220>
<221>  MOD_RES
<222>  (16)..(16)
<223>  PHOSPHORYLATION
<400>  15
Arg Val Ala Asp Pro Asp His Asp His Thr Gly Phe Leu Thr Glu Tyr
1               5                   10                  15

Val Ala Thr Arg Trp
            20

<210>  16
<211>  21
<212>  PRT
<213>  mus musculus

<220>
<221>  MOD_RES
<222>  (14)..(14)
<223>  PHOSPHORYLATION
<400>  16
Arg Val Ala Asp Pro Asp His Asp His Thr Gly Phe Leu Thr Glu Tyr
1               5                   10                  15

Val Ala Thr Arg Trp
            20

<210>  17
<211>  21
<212>  PRT
<213>  mus musculus

<220>
<221>  MOD_RES
<222>  (16)..(16)
<223>  PHOSPHORYLATION
<400>  17
Arg Val Ala Asp Pro Asp His Asp His Thr Gly Phe Leu Thr Glu Tyr
1               5                   10                  15

Val Ala Thr Arg Trp
            20

<210>  18
<211>  12
<212>  PRT
<213>  mus musculus

<220>
<221>  MOD_RES
<222>  (5)..(5)
<223>  PHOSPHORYLATION
<400>  18
Arg Ser Ser Met Ser Gly Leu His Leu Val Lys Gln
1               5                   10

<210>  19
<211>  12
<212>  PRT
<213>  mus musculus

<220>
<221>  MOD_RES
<222>  (3)..(3)
<223>  PHOSPHORYLATION

<400>  19

Arg Ser Ser Met Ser Gly Leu His Leu Val Lys Gln
1               5                   10

<210>  20
<211>  12
<212>  PRT
<213>  mus musculus

<220>
<221>  MOD_RES
<222>  (2)..(2)
<223>  PHOSPHORYLATION
<400>  20
Arg Ser Ser Met Ser Gly Leu His Leu Val Lys Gln
1               5                   10

<210>  21
<211>  21
<212>  PRT
<213>  mus musculus

<220>
<221>  MOD_RES
<222>  (16)..(16)
<223>  PHOSPHORYLATION
<400>  21
Arg Thr Pro Leu Ala Thr Ile Ala Asp Gln Gln Gln Leu Gln Leu Ser
1               5                   10                  15

Pro Leu Lys Arg Leu
            20